Europäisches Patentamt

European Patent Office

Office européen des brevets

(19)

(11) Publication number: **0 386 314**
**A1**

# EUROPEAN PATENT APPLICATION

(21) Application number: **89116887.4**

(51) Int. Cl.5: **A61B 5/00**

(22) Date of filing: **12.09.89**

(30) Priority: **03.03.89 JP 51671/89**

(43) Date of publication of application:
**12.09.90 Bulletin 90/37**

(84) Designated Contracting States:
**DE GB**

(71) Applicant: **FUKUDA DENSHI CO., LTD.**
**39-4, Hongo 3-chome Bunkyo-ku**
**Tokyo 113(JP)**

(72) Inventor: **Ohba, Kazuo Fukuda Denshi Co. Ltd.**
**Hongo enterprise place 2-35-8 Hongo**
**Bunkyo-ku Tokyo(JP)**

(74) Representative: **Behn, Klaus, Dipl.-Ing.**
**Patentanwalt Lindenberg 34**
**D-8134 Pöcking bei München(DE)**

(54) **System for monitoring a patient by using a LAN.**

(57) A system for monitoring a patient by using a LAN, comprising a first apparatus (2) for having no data and a second apparatus (3) for having data, which are mutually connected by means of a LAN (1). On the first apparatus (2), having no data, the indication of all the contents and the operation of all the settings of said second apparatus (3) having data, are able to be carried out.

# Fig. 1A

CENTRAL MONITOR ~2  FIRST APPARATUS FOR HAVING NO DATA

1  LAN

3A BEDSIDE MONITOR   3B BEDSIDE MONITOR   3C BEDSIDE MONITOR

3  SECOND APPARATUS FOR HAVING DATA

EP 0 386 314 A1

## BACKGROUND OF THE INVENTION

### 1. Field of the Invention

The present invention relates to a system for monitorring patient by using LAN. More particularly, it relates to a system for monitorring patient by using LAN, in which system an appayatus for having no data, as central monitor, and an apparatus for having data, as bedside monitor, are connected with by means of LAN.

### 2. Description of the Related Art

Generally, an apparatus for monitorring, that is to say, a monitor has been originally used in order to monitor always electrocardiogram etc. of a patient with an advanced disease accomodated in ICU, CCU etc..

However, the range of the use of the monitor has been recently increasing and, for example, the monitor is also used as that of a patient haused in general ward.

Accordingly, the monitor has become to be located as an apparatus necessary to clinical medicine.

The monitor is devided into a monitor for monitorring a single patient in bedside, that is to say, a bedside monitor, and a monitor for monitorring a great many patients in nurse station, that is say, a central monitor, and a system for monitorring patient is constituted by the former and the latter.

The bedside monitor has data, because electrocardiogram etc. of a patient is input into it.

On the contrary, the central monitor has no data, because it is setted in nurse station fay away from a patient and electrocardiogram etc. is directly not input into it.

In the conventional system for monitorring patient, the central monitor for having no data and the bedside monitor for having data are connected with by cable.

In this conventional system, the wave form of the living body signal is transmitted in analog, and the other control signal or numeral is transmitted in digital.

As aforementioned, the central monitor and the bedside monitor are connected with by cable.

However, the cable has originally the very low function, and since the cable transmits both analog and digital signals, the speed of treating dada, as the whole system, is late and one side direction communication is carried out.

Hence, in the prior art, on the side of the central monitor, all the contets of the bedside monitor cannot be observed and all the setting operations of the bedside monitor cannot be done.

That is to say, the prior art has the defect that the control of obsering all the contents and of doing all the setting operations, of the bedside monitor, cannot be respectively Oarried out, on the side of the central monitor.

## SUMMARY OF THE INVENTION

An object of the present invention is that all the control of the apparatus for having data can be carried out on the side of the apparatus for having no data, in the system for monitorring patient.

The above-mentioned object can be achieved by a system for monitorring patient by using LAN comprising a first apparatus for having no data and a second apparatus for having data which are mutually connected with by means of LAN, on the side of which first apparatus for having no data, the indication of all the contents and the operation of all the settings of said second apparatus for having data, are able to be carried out.

## BRIEF DESCRIPTION OF THE DRAWINGS

Further features and advantages of the present invention will be apparent from the ensuring description with reference to the accompanying drawings, wherein:

Fig. 1A is a drawing of the first embodiment of the present invention;

Fig. 1B is a drawing of the second embodiment of the present invention;

Fig. 1C is a drawing of the third embodiment of the present invention;

Fig. 2 is a detailed drawing of the embodiment of the present; invention;

Fig. 3 is a detailed drawing of the embodiment of the present invention.

## DESCRIPTION OF THE PREFERRED EMBODIMENS

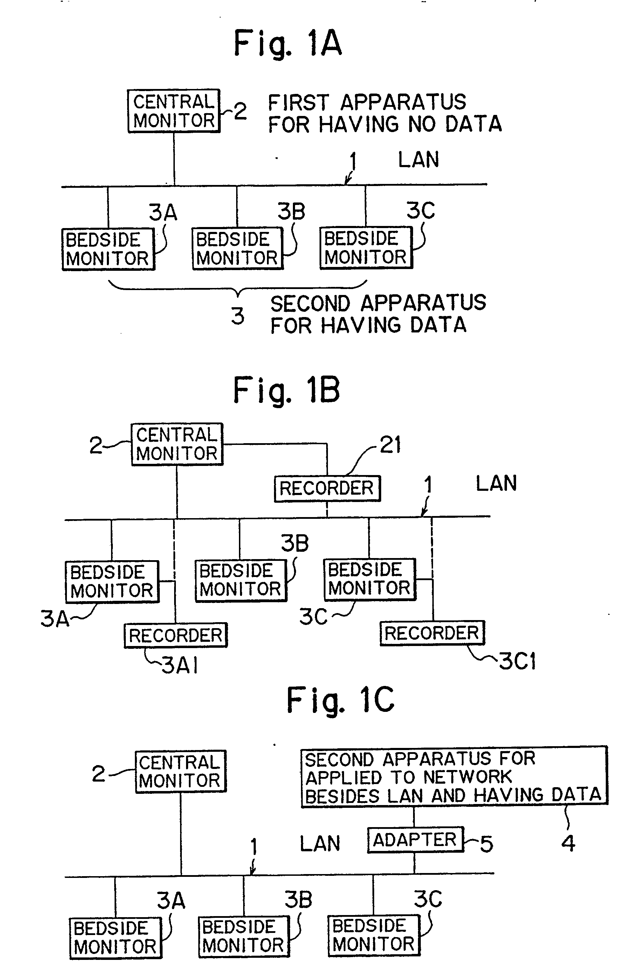

Figure 1A is a drawing of the first embodiment of the present invention, wherein reference numeral 1 shows LAN, 2 a first apparatus for having no data, 3 a second apparatus for having data.

Fig 1A is the case where a first apparatus 2 for having no data and a second apparatus 3 for having data are mutually commected with by means of LAN, on the side of which first apparatus 2 for having no data, the indication of all the contents and the operation of all the settings of said second apparatus 3 for having data, are able to be carried out.

There is, for example, a cental monitor, as the first apparatus 2 for having no data, and there is, for example, a bedside minitor, input box or receiver, as the second apparatus 3 for having data.

LAN 1 is short for Local Area Network, of which functionis about twice that of cable in the prior art and of which signal transmitting speed is about one hundred times that of cable in the prior art.

Moreover, the transmitting signal is the digtal signal and the mutual direction communication is capable of carried out, in LAN1, which is provide with the high speed communication IC characteristic of Local Area Network.

Hence, in the system for monitorring patient with the above constitution (see Fig.1A), allthe contents of the second apparatus 3 for having data is able to be observed, on the side of the first spparatus 2 for having no data.

The picture indicated in the second apparatus 3 for having data is the same as that in the first apparatus 2 for having no data.

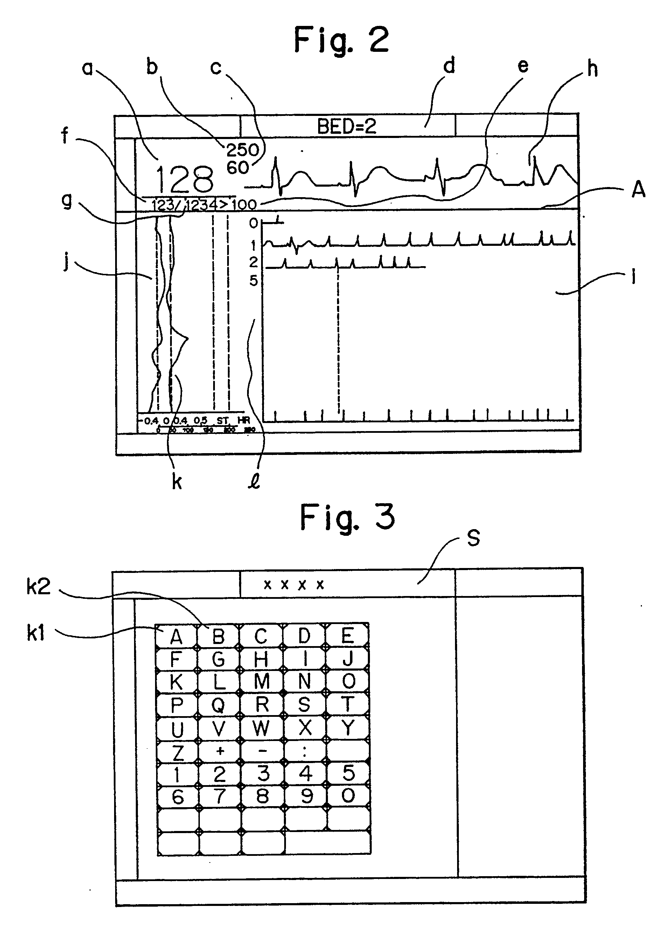

For example, the Σ indicating picture (see Fig.2) is indicated in the bedside monitor of the second apparatus 3 for having data,which picture is capable of being observed on the side of the central monitor of the first apparatus 2 for having data.

Σ indication picture of Fig.2 is the same as that of Fig.2 disclosed in Japanese Patent Application No. 1-27214 as filed on February 6,1989.

Generally, Σ indicating is defined as the indication of electrocardiogram in compression for a long time.

In Fig.2, Σ indicating pecture is indicated, which picture is that of the patient corresponding to the specific bed 2, as shown in a bed indication d.

With respect to boundary line A, in the down and upper portions, Σ indidicating pictures are respectively different.

That is to say, in the down portion, there is indicated the information regarding to electrocardiogram i in compression for about 3 minutes, in the upper portion, there is indicated the information regarding to erectrocardiogram h in compression for about 6 seconds.

In Fig. 2, reference numeral a shows an average heart beat, b the upper limit of the heart beat, c the lowest limit of the herrt beat, f the arrhythmia number per one minute, g the arrhythmia number per one hour, e the value smaller than the content of the above f, j the trend of ST, k an instant heart beat, 1 the arrhythmia number per each hour zone of the electrocardiogram i in compression for a long time.

The other pictures of the basic picture etc. indicated in the bedside monitor, is also able to be observed, on the side of the cental monitor.

The basic picture is indicated, when the condition of the patient is normally monitorred in this system.

On the other hand, the operation of all the settings of the second apparatus 3 for having data, is able to be carried out, on the side of the first apparatus 2 for having no data.

For example, the operation of setting (see Fig 3) the name of the patient in the bedside monitor 3A is able to be carried out, on the side of the central monitor 2.

Fig.3 shows the picture wherein the name of the patient is designated.

On the picture, key boards k1, k2 • • • are indicated and, when each key boards are pushed, the corresponding name is indicated in the upper portion S.

The indication of all the contents and the operation of all the settings, as aforementioned, are also capable of being carred out, between two second apparatuses for having data.

For example, when bedside monitors 3A, 3B and 3C are connected with to LAN 1 as shown in Fig. 1A, between any two bedside monitors, the indication of all the contents and the operation of all the settings of one monitor, are able to be carried out, on the side of the other monitor.

Figure 1B is a drawing of the second embodimene of the invention, which is, generally, the case where recorders are connected with both of the first apparatus 2 for having no data and the second apparatus 3 for having data, or with anyone of them, which recoders have for common use.

Especially, in Fig.1B, a recorder 21 is connected with the central monitor, as the first apparatus 2 for having no data, and recorders 3A1 and 3C1 are respectivly connected with bedside minitors 3A and 3C as the second appartuses 3 for having data.

Hence, for example, the recorder 3A1 connected with the bedside minitor 3A is able to be used on the side of the bedside monitor 3B, as if is connected with the bedside monitor 3B.

In Fig. 1B, the recorders 21, 3A1 and 3C1 are the same functions, which recoders may be directiy connected with LAN 1,as shown by dotted line.

Figure 1C is a drawing of the third embodiment of the invention, which is the case where a second apparatus 4 for applied to network besides LAN and for having data, is connected with LAN 1 through an adapter 5.

Hence, a system for monitorring patient constituted by the second apparatus 4 for having data, LAN 1, and the first apparatus 2 for having no data, has quite the same function as the system for monitorring patient constituted by the second apparatus 3 for having data, LAN 1, and the first apparatus 2 for having no data.

That is to say, the indication of all the contents and the operation of all the settings of the second apparatus 4 for having data, are able to be carried out, on the side of the first apparatus 2 for having no data.

According to the present invention, in a system for monitorring patient by using LAN, the first apparatus 2 for having no data and the second apparatus 3 for having data are mutually connected with by means of LAN, snd the indication of all the contents and the operation of all the settings of the seccond apparatus 3 for having data, are able to be oarried out, on the side of the first apparatus 2 for having no data.

LAN 1, compared with the cable of the prior art, transmits in digital, not only the control signal or numeral of the living body signal, but also the wave form of it, in which LAN the high speed and mutual direction communication may be done.

Therefore, since the system for monitorring patient is constituted by connecting the first apparatus 2 for having no data with the second apparatus 3 for having data through LAN 1, the indication etc. of all the contents of the second apparatus 3 for having data, are able to be carried out, on the side of the first appatus 2 for having no data.

This is to say, the present invention has the effect all the control operations of the apparatus for having data may be done, on the side of the apparatus for having no data.

5. A system for monitorring patient by using LAN according to claim 1, wherein said second apparatus (3) for having data is a bedside monitor.

6. A system for monitorring patient by using LAN according to claim 1, wherein said second apparatus (3) for haring data is an input box.

7. A system for monitorring patient by using LAN according to claim 1, wherein said second apparatus (3) for having data is a receiver.

8. A system for monitorring patient by using LAN according to claim 1, wherein, regarding to said first apparatus (2) for having no data and said second appararus (3) for having data, with both of them or anyone, recorders are connected, which recorders have for common use.

9. A systen for monitorring patient by using LAN according to claim 8, wherein said recorders have the same functions.

10. A systen for monitorring patient by using LAN according to claim 8, wherein said recorders are directy connected with LAN 1.

11. A system for monitorring patient by using LAN according to claim 1, wherein a second apparatus (4) for applied to nerwork besides LAN and for having data, is connected with LAN (1) through an adapter 5.

## Claims

1. A system for monitorring patient by using LAN comprising:
a first apparatus (2) for having no data and a second apparatus (3) for having date which are mutually connected with by means of LAN(1), on the side of which first apparatus (2) for having no data, the indication of all the contents and the operation of all the settings of said second apparatus (3) for having data, are able to be carried out.

2. A system for monitorring patient by using LAN according to claim 1, wherein the picture indicated in said second apparatus (3) for having data, is the same as that in said first apparatus (2) for having no data.

3. A system for monitorring patient by using LAN according to claim 1, wherein when a plurality of said second appatuses (3) for having data are connected with, between any two apparatuses, the indication of all the contents and the operation of all the settings of one monitor, are able to be carried out, on the side of the other monitor.

4. A system for monitorring patient by using LAN accoring to claim 1, wherein said first apparatu (2) for having no data is a central monitor.

## Fig. 1A

CENTRAL MONITOR ~2 — FIRST APPARATUS FOR HAVING NO DATA

1 — LAN

3A — BEDSIDE MONITOR

3B — BEDSIDE MONITOR

3C — BEDSIDE MONITOR

3 — SECOND APPARATUS FOR HAVING DATA

## Fig. 1B

2 — CENTRAL MONITOR

RECORDER — 21

1 — LAN

3A — BEDSIDE MONITOR

3B — BEDSIDE MONITOR

3C — BEDSIDE MONITOR

RECORDER — 3AI

RECORDER — 3C1

## Fig. 1C

2 — CENTRAL MONITOR

SECOND APPARATUS FOR APPLIED TO NETWORK BESIDES LAN AND HAVING DATA — 4

ADAPTER — 5

1 — LAN

3A — BEDSIDE MONITOR

3B — BEDSIDE MONITOR

3C — BEDSIDE MONITOR

# Fig. 2

a b c   d  e  h

BED=2

f 250
60
128

g 123/1234>100        A

j  0
1
2
5

  l

-0.4 0 0.4 0.5  ST  HR

k  l

# Fig. 3

       S

x x x x

k2
k1

| A | B | C | D | E |
| F | G | H | I | J |
| K | L | M | N | O |
| P | Q | R | S | T |
| U | V | W | X | Y |
| Z | + | - | : | |
| 1 | 2 | 3 | 4 | 5 |
| 6 | 7 | 8 | 9 | 0 |

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | Proc. of IEEE Eng. in Medicine & Biology Soc. 10th Annual Inter.Conference vol. 10, 04 November 88, pages 1112 - 1113; K.M.Reeves et al.: "Local Area Network (LAN) Protocol To Download Biosignals For Multiple Amplifier Sights Over A Single Medium" * the whole document *<br>--- | 1-11 | A61B5/00<br>G06F15/42 |
| Y | EP-A-269907 (HEWLETT-PACKARD COMPANY) * column 2, line 28 - column 4, line 55; figures 1-4 *<br>--- | 1-11 | |
| A | HEWLETT-PACKARD JOURNAL. vol. 31, no. 11, November 1980, PALO ALTO US pages 3 - 11; T.B.Blancke et al.: "Patient Monitoring Enhanced By New Central Station" * the whole document *<br>--- | 1-11 | |
| A | US-A-4804950 (J.B.MOON ET AL.) * column 2, line 37 - column 3, line 41 * * column 5, lines 12 - 30; figures 1, 2 *<br>----- | 1 | TECHNICAL FIELDS SEARCHED (Int. Cl.5)<br><br>A61B<br>G06F |

The present search report has been drawn up for all claims

| Place of search | Date of completion of the search | Examiner |
|---|---|---|
| THE HAGUE | 25 JUNE 1990 | HUNT B.W. |